# FASCICULE DE BREVET EUROPEEN

(11) **EP 0 425 398 B1**
(45) Date de publication et mention de la délivrance du brevet: **29.09.1993**
(21) Numéro de dépôt: 90430020.9
(22) Date de dépôt: 18.10.1990
(51) Int. Cl.: A61K 7/48, A61K 7/06

(54) **Eau d'aloès, procédé de préparation et compositions la renfermant**
Aloewasser, Herstellungsverfahren und Mittel, die dieses enthalten
Aloe solution, preparation process and compositions containing it

(30) Priorité: 23.10.1989 FR 8913832
(43) Date de publication de la demande: 02.05.1991
(73) Titulaire: Isnard, Camille, Noumea (Nouvelle Calédonie) (FR)
(72) Inventeur: Isnard, Camille, Noumea (Nouvelle Calédonie) (FR)
(74) Mandataire: Rinuy, Santarelli

(56) Documents cités:
- FR-A- 2 555 445
- US-A- 4 627 934
- PATENT ABSTRACTS OF JAPAN, vol. 9, no. 254 (C-308)[1977], 11 octobre 1985; & JP-A-60 109 526
- SEIFEN-ÖLE-FETTE-WACHSE, vol. 114, no. 1, 1988, pages 15-16,18-20, Augsburg, DE; R. RILK: "Zeitschrift für die Körperpflegemittel-, Parfümerie-, Riechstoff- u. Aerosol-Industrie"
- PATENT ABSTRACTS OF JAPAN, vol. 6, no. 169 (C-122)[1047], 2 septembre 1982; & JP-A-57 99 517
- Prospectos de Cosmêcal,concerant le produit "98" J Med Esth.et Chir. Derm.,vol XIX,74,juin92,129-133

## Description

La présente demande concerne une eau d'aloès, son procédé de préparation, son application, notamment en cosmétique et les compositions la renfermant.

En raison notamment de l'expansion constante des loisirs de plein air, on cherche toujours des produits protecteurs, voire réparateurs, de la peau. Ces produits devraient, en plus d'être efficaces, être bien tolérés, agréables d'emploi et d'odeur plaisante.

Par ailleurs, on recherche toujours des produits actifs dans la lutte contre la calvitie, soit pour retarder celle-ci, soit pour favoriser la repousse des cheveux.

Il est déjà connu par FR-A-2.555.445 un gel d'aloès doué de propriétés kératolytiques, antiseptiques, hydratantes, cicatrisantes et calmantes.

US-A-4.627.934 décrit pour sa part une composition hydro-alcoolique à base d'une fraction organique de distillat d'aloès, à propriétés calmantes mises à profit dans un après-rasage.

La demanderesse a découvert avec étonnement qu'une eau d'aloès avait de remarquables propriétés, tant protectrices et réparatrices de la peau que pour combattre la calvitie.

C'est pourquoi la présente demande a pour objet une eau d'aloès, caractérisée en ce qu'elle est susceptible d'être obtenue par condensation de la vapeur d'ébullition d'un broyat de feuilles d'aloès. L'aloès peut appartenir à toute espèce connue, et en particulier, par exemple à l'espèce Aloe barbadensis. Dans certaines applications, on a découvert que l'eau d'aloès ci-dessus avait des propriétés améliorées lorsque le broyat était soumis à une fermentation, préalablement à l'opération d'ébullition en vue de la condensation de la vapeur. C'est pourquoi la présente demande a également pour objet une eau d'aloès ci-dessus décrite caractérisée en ce que l'ébullition du broyat est précédée par une fermentation dudit broyat.

La présente demande a aussi pour objet un procédé de préparation de l'eau d'aloès ci-dessus décrite caractérisé en ce que l'on soumet à l'ébullition un broyat de feuilles d'aloès, si désiré après fermentation du broyat et condense la vapeur pour obtenir le produit attendu. Le broyat de feuilles d'aloès peut être préparé comme suit. On ramasse les feuilles d'aloès de préférence Aloe barbadensis en coupant des feuilles d'une longueur de 40 à 50 cm environ ; on les lave, les sèche puis les passe dans un broyeur, par exemple à couteaux, pour obtenir un broyat homogène. L'ébullition du broyat est menée de préférence dans un bouilleur capable d'être hermétiquement clos et comportant notamment un indicateur de température et de pression. L'ébullition sera réalisée de préférence sous pression réduite, notamment à une pression d'environ 1330 Pa (10 mm de mercure). La vapeur est ensuite envoyée par exemple dans un condenseur en verre ou toute autre matière adaptée refroidi par exemple à l'air et notamment à l'eau. La récupération de l'eau d'aloès attendue se fera de préférence dans un récipient stérile.

Dans des conditions préférentielles de mise en oeuvre du procédé ci-dessus décrit, préalablement à l'opération d'ébullition, le broyat est mis à fermenter pendant une durée allant de 6 à 48 heures mais de préférence d'environ 24 heures.

La présente invention a bien évidemment également pour objet l'eau d'aloès obtenue directement par la mise en oeuvre du procédé ci-dessus décrit.

L'eau d'aloès ci-dessus décrite a de remarquables propriétés tant dans le traitement de la peau que dans le traitement de la calvitie.

C'est pourquoi la présente demande a également pour objet les compositions cosmétiques caractérisées en ce qu'elles renferment une eau d'aloès telle que définie ci-dessus.

La présente invention a plus particulièrement comme objet les compositions cosmétiques caractérisées en ce qu'elles sont destinées à un traitement capillaire et également celles destinées au traitement de la peau. A cette fin, l'eau d'aloès ci-dessus décrite peut être mélangée à des préparations liquides ou des crèmes, à des émulsions, par exemple hydrophi- i-les. On peut également réaliser, à partir de l'eau d'aloès ci-dessus décrite et à l'aide de surfactifs convenables bien connus de l'homme de l'art, des émulsions notamment huile dans l'eau. On peut également préparer des lotions aqueuses ou alcooliques qui renfermeront notamment un alcool, particulièrement un alcool lourd tel qu'un glycol, le glycérol ou un de ses dérivés.

L'eau d'aloès selon l'invention peut être mélangée si désiré à d'autres produits actifs de manière à améliorer son efficacité.

C'est pourquoi la présente demande a aussi pour objet un produit à base d'eau d'aloès caractérisée en ce qu'il renferme, en outre de l'eau d'aloès, un broyat de la partie solide séchée du broyat ci-dessus décrit après traitement à ébullition, des fleurs rouges d'hibiscus, des feuilles de goyave, du jus de citron et de l'essence de citronnelle.

Un produit préféré selon l'invention renferme, pondéralement, par rapport à l'eau d'aloès,
- environ 0,005 %, par exemple 0,02 à 0,001 et notamment 0,01 à 0,0025 % dudit broyat de la partie solide séchée du broyat de feuilles d'aloès après l'opération d'ébullition,
- environ 0,012 %, par exemple de 0,05 à 0,003 et de préférence de 0,025 à 0,006 % de fleurs rouges déshydratées d'hibiscus,
- environ 0,0025 %, par exemple 0,01 à 0,0006 % et notamment, de 0,005 à 0,001 % de feuilles de goyave déshydratées,
- environ 0,005 %, par exemple 0,02 à 0,001 % et de préférence 0,01 à 0,0025 % de jus de citron et,
- environ 0,000015 %, par exemple de 0,00006 à 0,000003 %, de préférence 0,00003 à 0,000007 % d'essence de citronnelle.

Un produit particulièrement préféré à base d'eau d'aloès selon la présente invention renferme pondéralement 0,12 % de fleurs rouges déshydratées d'hibiscus, 0,0025 % de broyat de la partie solide séchée d'un broyat de feuilles d'aloès, après opération d'ébullition, 0,0025 % de feuilles de goyave déshydratées, 0,005 % de jus de citron et 0,000015 % d'essence de citronnelle par rapport à l'eau d'aloès.

L'Hibiscus peut être notamment Hibiscus rosa sy- nensis L.; la goyave utilisée est notamment Psidium guajava L.; le jus de citron est de préférence clarifié, notamment par filtration et provient, en particulier, de Citrus limon Burmann ; la citronnelle appartient notamment à l'espèce Cymbopogon citratus (DC Stapf).

Les produits ci-dessus décrits peuvent être préparés notamment en ajoutant en décoction à l'eau d'aloès ci-dessus décrite, les feuilles d'hibiscus, les feuilles de goyave, le broyat du résidu solide de feuilles d'aloès, le jus de citron, l'essence de citronnelle, laissant macérer, séparant du résidu solide le liquide qu'on laisse fermenter, puis séparant le nouveau résidu solide pour obtenir le produit attendu sous forme de liquide, et en ajoutant, si désiré, le ou les additifs.

Les fleurs rouges d'hibiscus mises en oeuvre sont de préférence lavées et déshydratées.

Les feuilles de goyave sont notamment lavées, déshydratées et broyées.

Le jus de citron peut être utilisé tel quel mais est avantageusement clarifié, notamment par filtration.

Lorsque les différents constituants sont mis en présence, on laisse alors macérer l'ensemble dans une cuve, de préférence environ 72 heures, lorsque l'on opère à température ambiante. Cette durée peut être diminuée si l'on opère un léger chauffage.

La séparation ultérieure des résidus peut être réalisée selon les techniques bien connues, telles que la centrifugation, et notamment la filtration.

Le liquide ainsi récupéré estalors mis à fermenter pendant environ 4 mois, notamment de 3 à 5 mois, selon la qualité et la quantité des éléments mis en oeuvre. Lorsque la teneur en tanins de l'hibiscus est importante, un temps de fermentation plus long est préféré.

La séparation ultérieure peut également être pratiquée comme indiqué ci-dessus, notamment par filtration.

Le produit ainsi obtenu peut être utilisé tel quel mais il est possible de lui adjoindre des additifs tels que des antioxydants, comme l'acide ascorbique, par exemple à raison de 30 mg/litre, des antibactériens, telle que l'eau de Dalibour, à raison, dans ce cas, de 3 ml/litre, ou d'autres conservateurs.

Les produits destinés à une application cutanée ci-dessus peuvent être mélangés à des préparations liquides ou crèmes de bronzage, ou à une émulsion par exemple hydrophile. On peut également réaliser, à partir de tels produits et l'aide de suractifs convenables bien connus de l'homme de l'art, des émulsions huile dans l'eau.

On peut également préparer des lotions qui renfermeront notamment un alcool particulièrement un alcool lourd, tel qu'un glycol, le glycérol ou l'un de ses dérivés.

Il est bien entendu possible d'adjoindre, selon les besoins, d'autres additifs tels que des produits parfumants, de préférence hypoallergéniques, des colorants ou des filtres notamment solaires.

Les produits selon la présente invention ontde remarquables propriétés ; notamment ils permettent l'hydratation de la peau, sont calmants, adoucissants, anti- inflammatoires; utilisés à titre préventif, ils évitent la formation de cloques malgré une exposition prolongée au soleil ; ils permettent par ailleurs de prolonger le bronzage, notamment pour les types de peaux sensibles, par exemple peau blanche de blonds ou de roux, ainsi que les peaux sensibles en général, particulièrement les peaux de bébés.

Les produits selon la présente invention peuvent être utilisés en traitement préventif vis-à-vis du soleil et du vent, grâce notamment à leur action hydratante évitant le dessèchement de la peau et également à titre curatif, par exemple après une exposition prolongée au soleil.

Les produits selon la présente invention possèdent également de remarquables propriétés cicatrisantes.

Les produits selon la présente invention permettent en outre de calmer presque instantanément les effets douloureux de l'érythème solaire et, utilisés rapidement, permettent de limiterfortement l'apparition de cloques sur la peau.

Les produits de l'invention ne sont pas gras et, de ce fait, permettent de se vêtir aussitôt après leur application. Ils présentent également l'avantage en raison de leur fluidité, de pouvoir être conditionnés en vaporisateur, en atomiseur ou en microniseur, permettant ainsi d'éviter une application douloureuse, étant donné qu'un massage n'est pas nécessaire pour permettre leur pénétration. En effet, les produits ci-dessus décrits sont aisément absorbés par l'épiderme.

La présente demande a tout autant pour objet un produit à base d'eau d'aloès caractérisé en ce qu'il renferme, en outre de l'eau d'aloès, du camphre, du sulfate de cuivre, et de la triéthanolamine.

Dans des conditions préférentielles, le produit ci-dessus décrit renferme pondéralement, par rapport à l'eau d'aloès, environ 0,05 % de camphre, par exemple 0,2 à 0,01 %, et notamment de 0,2 à 0,025 % ; environ 0,005 % de sulfate de cuivre, par exemple 0,01 à 0,0006 % et notamment 0,006 à 0,001 % dudit sulfate de cuivre, environ 0,2 % de triéthanolamine, par exemple 0,5 % à 0,01 % et notamment de 0,3 à 0,1 % et environ 0,1 % d'acide lactique, par exemple de 0,5 à 0,01 % et notamment de 0,3 à 0,05 %.

Le sulfate de cuivre est avantageusement utilisé sous forme de son pentahydrate. On peut utiliseréga- lement d'autres sels de cuivre divalents, non toxiques.

Un produit préféré selon l'invention renferme en outre de l'acide lactique. Un produit tout particulièrement préféré selon l'invention a la composition suivante donnée pour litre d'eau d'aloès selon la présente invention :
Camphre 0,05 %, sulfate de cuivre pentahydraté 375 mg/1, éthanol 0,3 %, triéthanolamine 0,238 %, acide lactique 0,0952 %. Le pH de la composition s'établit à 7,5.

Le produit ci-dessus décrit trouve de remarquables applications dans le traitement de la calvitie. Il est en effet capable de diminuer la séborrhée, de réactiver les cellules capillaires et de stopper la chute des cheveux. On constate également, dans la plupart des cas, une stimulation de la croissance des kératinocy- tes et la pousse des cheveux. On a également pu constater une modification de la nature du cheveu, celui-ci devenant plus épais.

Le produit ci-dessus décrit peut être utilisé comme suit : on opère deux applications par jour, par exemple matin et soir, notamment sur des cheveux propres et secs. On peut effectuer les applications raie par raie, à la racine des cheveux, depuis le sommet du crâne et en massant le cuir chevelu pour permettre la pénétration du produit. Il est également souhaitable de ne pas rincer les cheveux après utilisation.

Les produits ci-dessus décrits sont remarquablement tolérés. En effet, aucune réaction d'irritation, d'intolérance ou de sensibilisation n'a été observée au cours des différentes études cliniques, même au long cours. Les propriétés des produits de la présente demande sont illustrées ci-après dans la partie expérimentale.

### Exemple 1

On ramasse les feuilles d'Aloe barbadensis en coupant des feuilles d'une longueur de 40 à 50 cm environ, les lave, les sèche, et les broie dans un broyeur à couteaux pour obtenir un broyat homogène. On fait alors fermenter pendant 24 h. Après la fermentation, on place dans un bouilleur d'une capacité de 150 1 hermétiquement clos comportant des indications de température et de pression et une sortie d'un diamètre de 3 cm pour la récupération de la vapeur. La vapeur est envoyée dans un condenseur en verre refroidi à l'eau. On opère à ébullition, sous pression réduite, à 10 mm de mercure. On obtient le produit attendu avec un rendement de 65 % environ. Le rendement de préparation est d'environ 4 à l'heure. 150 Kg de feuilles d'aloès mis en oeuvre produisent ainsi 97 1 d'eau d'aloès selon l'invention en 24 h appelée ci-après P2. On récupère le broyat solide, le sèche et le broie pour obtenir un produit dénommé ci-après P3.

Le jus (P2) est incolore, translucide et présente une densité de 0,998.

On a également préparé une eau d'aloès P'2 par broyage de feuilles. La partie solide du broyat séchée et broyée est appelée P'3.

Par ailleurs, on lave et déshydrate les fleurs rouges d'Hibiscus rosa sinensis L., déshydrate et broie les feuilles de Psidium guajava. On place dans une cuve 30 litres de P2, ajoute en décoction, à 20°C environ, 0,012 % de fleurs d'hibiscus, 0,0025 % de feuilles de goyave, 0,005 % de P3, 0,005 % de jus de citron filtré et 0,000015 % d'essence de citronnelle.

On effectue une préparation similaire à partir de P'2 et de P'3. On laisse pendant 72 heures à 20°C environ, filtre la décoction, place le filtrat dans un fermenteur pendant 100 jours à 30°C environ sous une pression de 150 mm de colonne d'eau et filtre pour obtenir les produits désirés, d'aspect translucide brun-rouge, d'odeur légèrement fruitée et acidulée et de pH 5,5.

### Exemple 2

A un litre des produits obtenus à l'exemple 1, on ajoute 3 ml d'eau de Dalibour et 30 mg d'acide ascorbique pour obtenir des préparations de longue conservation.

### Exemple 3 : lait dermatologique

On a préparé des laits dermatologiques ayant la composition suivante par flacon :
- produits de l'exemple 1 70 ml
- stéarate de glycérol ; cétostéarate de po- lyoxyéthylène ; isononanoate de stéaryle ; glycérol ; acide lactique ; triéthanolamine 30 ml

### Exemple 4: lotion non alcoolique

On a conditionné en flacon aérosol 200 ml des produits de l'exemple 1.

### Exemple 5 : crème

On a préparé des crèmes comprenant :
- produits de l'exemple 1 70 ml
- stéarate de glycérol ; cétostéarate de po- lyoxyéthylène ; di-n-bytyl-adipate ; huile de parafine ; glycérol ; acide lactique ; tri-éthano- lamine 30 ml

### Exemple 6 : gel

On a préparé des gels répondant à la formule suivante :
- produits de l'exemple 1 50 ml
- monopropylèneglycol ; acide poly-acrylique réticulé ; acide lactique ; triéthanolamine 50 ml

### Exemple 7 : essai d'efficacité des produits de l'exemple 1

A) On a traité une brûlure au 3ème degré s'étendant sur une surface de 12 cm². Les produits de l'exemple 2 ont été appliqués directement sur la brûlure. On a renouvelé l'application toutes les deux heures, sans couvrir la brulure. Le lendemain, on a procédé également à trois applications.

On a observé, 30 minutes après la première application, un soulagement pratiquement complet de la douleur. Le lendemain de cette première application, la brulure était recouverte d'une fine croûte nette, sans suintement ni infection apparente.

Les jours 3, 4 et 5, on a procédé à deux applications par jour à l'aide d'une compresse stérile. Après trois semaines, la peau était nette et lisse, sans cicatrices ou boursouflures.

B) Les produits de l'exemple 1 ont été utilisés sur dix sujets souffrant de brûlures au premier degré, après exposition prolongée aux ultraviolets. La plupart présentaient une douleur au toucher et une difficulté de bouger les membres concernés sans ressentir une vive douleur, compte tenu de l'importance de l'érythème.

Les peaux étaient de plusieurs types, notamment peau de sujet roux.

Deux heures après la fin de l'exposition, le produit à tester a été appliqué. On a constaté un soulagement immédiat de la douleur, la récupération non douloureuse des mouvements, de même qu'une sensation de fraîcheur. Le toucher n'était plus douloureux, de même que le frottement.

On a également observé que le hâle obtenu par l'exposition aux ultraviolets était conservé plus longtemps chez des sujets ayant bénéficié d'un traitement avec le produit de la présente invention, par rapport à des sujets n'en ayant pas bénéficié.

Les produits préparés à partir de P2 et de P3 se sont avérés plus actifs que ceux préparés à partir de P'2 et P'3.

### Exemple 8 : Lotion capillaire

### a) Préparation du sulfate de cuivre

On dissout 29 g de sulfate de cuivre pentahydraté pur à 99 % dans de 230 ml d'eau distillée à 25 °C, ce qui correspond à une teneur en sulfate de cuivre de 12,6 % par rapport au liquide.

### b) Préparation de la triéthanolamine et de l'acide lactique

On dissout 175 ml de triéthanolamine et 70 ml d'acide lactique dans 524 ml d'eau distillée.

### c) Préparation d'une lotion aqueuse destinée à traiter la calvitie

On mélange 97 1 d'eau d'aloès (P2) avec 230 ml de préparation de sulfate de cuivre ci-dessus, 524 ml de préparation triéthanolamine-acide lactique ci-dessus et 1,5 1 d'alcool camphré pour obtenir le produit attendu.

Le produit de l'exemple 8 réactive les cellules capillaires et stoppe la chute des cheveux.

## Revendications (Revendications pour l'(les) Etat(s) contractant(s) suivant(s) : suivants: CH, DE, FR, GB, IT, LI)

1. Eau d'aloès, caractérisée en ce qu'elle est susceptible d'être obtenue par condensation de la vapeur d'ébullition d'un broyat de feu illes d'aloès.

2. Eau d'aloès selon la revendication 1, caractérisée en ce que l'ébullition du broyat est précédée par une fermentation dudit broyat.

3. Procédé de préparation de l'eau d'aloès selon la revendication 1 ou 2, caractérisé en ce que l'on soumet à l'ébullition un broyat de feuilles d'aloès, sidésiré après fermentation du broyat, et condense la vapeur pour obtenir le produit attendu.

4. Procédé selon la revendication 3, caractérisé en ce que l'ébullition est réalisée sous pression réduite.

5. Procédé de préparation du produit selon l'une quelconque des revendications 3 ou 4, caractérisé en ce que le broyat est fermenté pendant une durée d'environ 24 heures, préalablement à l'ébullition.

6. Composition cosmétique caractérisée en ce qu'elle renferme une eau d'aloès selon la revendication 1 ou 2.

7. Composition selon la revendication 6 caractérisée en ce qu'elle est destinée à un traitement capillaire.

8. Composition cosmétique selon la revendication 7 caractérisée en ce qu'elle renferme du camphre, du sulfate de cuivre et de la triéthanolamine et de l'acide lactique.

9. Composition selon la revendication 6, caractérisée en ce qu'elle destinée au traitement de la peau.

10. Composition selon la revendication 9 caractérisée en ce qu'elle renferme, outre l'eau d'aloès, un broyat de la partie solide d'un broyat de feuilles d'aloès, après traitement à ébullition, de fleurs d'hibiscus, de feuilles de goyaves, du jus de citron et de l'essence de citronelle.

## Revendications (Revendications pour l'(les) Etat(s) contractant(s) suivant(s) : suivant: ES)

1. Procédé de préparation d'eau d'aloès caractérisé en ce que l'on soumet à l'ébullition un broyat de feuilles d'aloès, si désiré après fermentation du broyat, et condense la vapeur pour obtenir le produit attendu.

2. Procédé selon la revendication 1, caractérisé en ce que l'ébullition du broyat est précédée par une fermentation dudit broyat.

3. Procédé selon la revendication 1 ou 2, caractérisé en ce que l'ébullition est réalisée sous pression réduite.

4. Procédé de préparation de l'eau d'aloès selon l'une quelconque des revendications 2 ou 3, caractérisé en ce que le broyat est fermenté pendant une durée d'environ 24 heures, préalablement à l'ébullition.

5. Composition cosmétique caractérisée en ce qu'elle renferme une eau d'aloès obtenue à l'aide du procédé selon l'une des revendications 1 à 4.

6. Composition selon la revendication 5 caractérisée en ce qu'elle est destinée à un traitement capillaire.

7. Composition cosmétique selon la revendication 6 caractérisée en ce qu'elle renferme du camphre, du sulfate de cuivre et de la triéthanolamine et de l'acide lactique.

8. Composition selon la revendication 5, caractérisée en ce qu'elle destinée au traitement de la peau.

9. Composition selon la revendication 8 caractérisée en ce qu'elle renferme, outre l'eau d'aloès, un broyat de la partie solide d'un broyat de feuilles d'aloès, après traitement à ébullition, de fleurs d'hibiscus, de feuilles de goyaves, du jus de citron et de l'essence de citronelle.

## Patentansprüche (Patentansprüche für folgende(n) Vertragsstaat(en) : CH, DE, FR, GB, IT, LI)

1. Aloewasser, dadurch gekennzeichnet, daß es durch Kondensation des Siededampfes fein zerkleinerter Aloeblätter erhalten werden kann.

2. Aloewasser nach Anspruch 1, dadurch gekennzeichnet, daß man vor dem Sieden des Zerkleinerungsgutes eine Fermentation desselben durchführt.

3. Verfahren zur Herstellung des Aloewassers gemäß Anspruch 1 oder 2, dadurch gekennzeichnet, daß man fein zerkleinerte Aloeblätter, falls gewünscht, nach deren Fermentation, zum Sieden erhitzt und den Dampf zur Erzielung des gewünschten Produktes kondensiert.

4. Verfahren nach Anspruch 3, dadurch gekennzeichnet, daß das Sieden unter vermindertem Druck erfolgt.

5. Verfahren zur Herstellung des Produktes nach irgendeinem der Ansprüche 3 oder 4, dadurch gekennzeichnet, daß das Zerkleinerungsgut vor dem Sieden für eine Dauer von etwa 24 h fermentiert wird.

6. Kosmetische Zusammensetzung, dadurch gekennzeichnet, daß sie ein Aloewasser gemäß Anspruch 1 oder 2 umfaßt.

7. Zusammensetzung nach Anspruch 6, dadurch gekennzeichnet, daß sie für die Haarbehandlung beabsichtigt ist.

8. Kosmetische Zusammensetzung nach Anspruch 7, dadurch gekennzeichnet, daß sie Kampfer, Kupfersulfat und Triethanolamin sowie Milchsäure umfaßt.

9. Zusammensetzung nach Anspruch 6, dadurch gekennzeichnet, daß sie für die Behandlung der Haut beabsichtigt ist.

10. Zusammensetzung nach Anspruch 9, dadurch gekennzeichnet, daß sie außer dem Aloewasser Zerkleinerungsgut des festen Anteils eines Zerkleinerungsgutes der Aloeblätter nach der Siedebehandlung, Hibiskusblätter, Guavablätter, Zitronensaft und Citronellessenz enthält.

## Patentansprüche (Patentansprüche für folgende(n) Vertragsstaat(en) : ES)

1. Verfahren zur Herstellung von Aloewasser, dadurch gekennzeichnet, daß man fein zerkleinerte Aloeblätter, falls gewünscht, nach deren Fermentation, zum Sieden erhitzt und den Dampf zur Erzielung des gewünschten Produktes kondensiert.

2. Verfahren nach Anspruch 1, dadurch gekennzeichnet, daß man vor dem Sieden des Zerkleinerungsgutes eine Fermentation desselben durchführt.

3. Verfahren nach Anspruch 1 oder 2, dadurch gekennzeichnet, daß das Sieden unter vermindertem Druck erfolgt.

4. Verfahren zur Herstellung des Aloewassers nach irgendeinem derAnsprüche 2 oder 3, dadurch gekennzeichnet, daß das Zerkleinerungsgut vor dem Sieden für eine Dauer von etwa 24 h fermentiert wird.

5. Kosmetische Zusammensetzung, dadurch gekennzeichnet, daß sie ein Aloewasser umfaßt, das nach dem Verfahren gemäß Anspruch 1 bis 4 erhalten wurde.

6. Zusammensetzung nach Anspruch 5, dadurch gekennzeichnet, daß sie für die Haarbehandlung beabsichtigt ist.

7. Kosmetische Zusammensetzung nach Anspruch 6, dadurch gekennzeichnet, daß sie Kampfer, Kupfersulfat und Triethanolamin sowie Milchsäure umfaßt.

8. Zusammensetzung nach Anspruch 5, dadurch gekennzeichnet, daß sie für die Behandlung der Haut beabsichtigt ist.

9. Zusammensetzung nach Anspruch 8, dadurch gekennzeichnet, daß sie außer dem Aloewasser Zerkleinerungsgut des festen Anteils eines Zerkleinerungsgutes der Aloeblätter nach der Siedebehandlung, Hibiskusblätter, Guavablätter, Zitronensaft und Citronellessenz enthält.

## Claims (Claims for the following Contracting State(s) : CH, DE, FR, GB, IT, LI)

1. Aloe water, characterized in that it is capable of being obtained by condensing the ebullition vapour of a pulp of aloe leaves.

2. Aloe water according to claim 1, characterized in that the boiling of the pulp is preceded by a fermentation of said pulp.

3. Preparation process for aloe water according to claim 1 or 2, characterized in that a pulp of aloe leaves is subjected to boiling, if desired after fermentation of the pulp, and the vapour is condensed in order to obtain the expected product.

4. Process according to claim 3, characterized in that the boiling is carried out under reduced pressure.

5. Preparation process of the product according to any one of claims 3 or 4, characterized in that the pulp is fermented for a duration of about 24 hours, before boiling.

6. Cosmetic composition characterized in that it contains aloe water according to claim 1 or 2.

7. Composition according to claim 6 characterized in that it is intended for hair treatment.

8. Cosmetic composition according to claim 7 characterized in that it contains camphor, copper sulphate and triethanolamine and lactic acid.

9. Composition according to claim 6 characterized in that it is intended for skin treatment.

10. Composition according to claim 9, characterized in that it contains, in addition to aloe water, a pulp of the solid part of a pulp of aloe leaves, after treatment by boiling, of hibiscus flowers, guava leaves,lemon juice and citronella essence.

## Claims (Claims for the following Contracting State(s) : ES)

1. Preparation process for aloe water, characterized in that a pulp of aloe leaves is subjected to boiling, if desired after fermentation of the pulp, and the vapour is condensed in order to obtain the expected product.

2. Process according to claim 1, characterized in that the boiling of the pulp is preceded by a fermentation of said pulp..

3. Process according to claim 1 or 2, characterized in that the boiling is carried out under reduced pressure.

4. Preparation process for aloe water according to any one of claims 2 or 3, characterized in that the pulp is fermented for a duration of about 24 hours, before boiling.

5. Cosmetic composition characterized in that it contains aloe water obtained using the process according to one of claims 1 to 4.

6. Composition according to claim 5, characterized in that it is intended for hair treatment.

7. Cosmetic composition according to claim 6, characterized in that it contains camphor, copper sulphate and triethanolamine and lactic acid.

8. Composition according to claim 5, characterized in that it is intended for skin treatment.

9. Composition according to claim 8, characterized in that it contains, in addition to aloe water, a pulp of the solid part of a pulp of aloe leaves, after treatment by boiling, hibiscus flowers, guava leaves, lemon juice and citronella essence.
